# EUROPEAN PATENT APPLICATION

(11) **EP 4 478 370 A1**
(43) Date of publication of application: **18.12.2024**
(21) Application number: 23179140.1
(22) Date of filing: 14.06.2023
(51) Int. Cl.: G16H 10/60, G16H 50/30, G16H 50/70, G16H 50/20

(54) **GENERATING AN AMERICAN SOCIETY OF ANESTHESIOLOGISTS PHYSICAL STATUS SCORE**

(71) Applicant: Koninklijke Philips N.V., 5656 AG Eindhoven (NL)
(72) Inventor: ARORA, Ashima, 5656 AG Eindhoven (NL)
(74) Representative: Philips Intellectual Property & Standards

(57) **Abstract**

A mechanism for predicting an ASA-PS score for a medical subject. Medication information of the subject is processed using a machine-learning method to predict the ASA-PS score.

## Description

### FIELD OF THE INVENTION

The present invention relates to the field of medical scoring.

### BACKGROUND OF THE INVENTION

The American Society of Anesthesiologists Physical Status (ASA-PS) classification system is an approach for simple categorization of a medical subject's pre-anesthesia medical co-morbidities.

A clinician using the ASA-PS classification system on a medical subject will produce an ASA-PS score that effectively defines or categorizes the fitness of a medical subject before a treatment requiring anesthesia, e.g., surgery. The ASA-PS score has proven to be a reliable independent predictor of medical complications, perioperative risks and mortality following surgery.

Alternative labels for an ASA-PS score include an ASA-PS classification, an ASA-PS category and/or an ASA-PS value. These terms may be used interchangeably.

Generally, the ASA-PS score will identify the medical subject as belonging to up to six categories labelled 1 to 6. These categories are typically characterized as: (1) Healthy person; (2) Mild systemic disease; (3) Severe systemic disease; (4) Severe systemic disease that is a constant threat to life; (5) a moribund person who is not expected to survive without treatment (e.g., the surgery); and (6) declared brain-dead person whose organs are being removed for donor purposes. It is known for category (6) to be omitted in some use-case scenarios.

However, it is common for the ASA-PS score for a medical subject to go unreported/unrecorded, e.g., it may not be used in all clinical settings (e.g., hospitals etc.). Absence of the ASA-PS score may have significant implications for the medical subject for future treatment, e.g., post-surgical treatment and/or recovery. Moreover, accurate generation of an ASA-PS score places a significant clinical burden on clinicians.

There is therefore a desire for an automated mechanism for determining the ASA-PS score of a medical subject.

### SUMMARY OF THE INVENTION

The invention is defined by the claims.

According to examples in accordance with an aspect of the invention, there is provided a computer-implemented method for predicting an American Society of Anesthesiologists Physical Status, ASA-PS, score for a medical subject.

The computer-implemented method comprises obtaining medication information identifying any medications prescribed and/or taken by the medical subject; and processing at least the medication information using a machine-learning method to predict the ASA-PS score for the medical subject.

The present disclosure recognizes that there is a link or correlation between any medications taken by a medical subject and their ASA-PS score. In particular, the number, type and/or identity of medications taken by the medical subject has been linked with an increased likelihood of co-morbidities in the medical subject, and therefore with a higher ASA-PS score. The present invention proposes the use of a suitably trained machine-learning method to process at least medication information of the medical subject in order to predict (e.g., output) an ASA-PS score for the medical subject.

A model that is able to predict ASA-PS scores can prove helpful in classifying patient severity, predict perioperative and postoperative complications, predict resources a subject might need in the hospital and would give overall more accurate results.

An ASA-PS score represents a physical state of the medical subject. The proposed approach provides a technique for determining the ASA-PS score based on other information about the medical subject, thereby providing a mechanism for determining the ASA-PS score from indirect information.

The computer-implemented method may further comprise obtaining demographic information of the medical subject, wherein processing at least the medication information comprises processing at least the demographic information and the medication information using the machine-learning method to predict the ASA-PS score.

Embodiments recognize that there is a causal link between demographics that affects the likely co-morbidities of a medical subject, and therefore the ASA-PS score of the medical subject. Thus, a more accurate prediction of the ASA-PS score can be achieved by further taking demographic information into account when producing the ASA-PS score.

In some examples, the demographic information comprises at least an age and/or gender of the medical subject. These embodiments recognize that there is a strong correlation or link between age/gender and ASA-PS score. Thus, improved accuracy in determining the ASA-PS score can be achieved by using age and/or gender information in calculating the ASA-PS score.

Optionally, the computer-implemented method further comprises obtaining renal state information indicating a renal state functionality of the medical subject, wherein processing at least the medication information comprises processing at least the renal state information and the medication information using the machine-learning method to predict the ASA-PS score. These embodiments recognize that there is a strong correlation or link between renal state functionality and ASA-PS score. Thus, improved accuracy in determining the ASA-PS score can be achieved by using such information in calculating the ASA-PS score.

The renal state information may identify a change from normal functionality of the renal system of the medical subject.

In some examples, the medication information comprises at least one Anatomical Therapeutic Chemical, ATC, code for the medical subject, each ATC code identifying a medication prescribed and/or taken by the medical subject. This approach ensures reliable and transferable information on the medication information is used. In particular, the ATC codes are structured in a known format, which facilitates ease of direct inputting into the machine-learning method.

In some examples, the step of processing at least the medication information using the machine-learning method comprises: processing each ATC code to identify one or more categories of medication prescribed to and/or taken by the medical subject; and processing the identified categories of medication using the machine-learning method to predict the ASA-PS score for the medical subject.

In some examples, each category is defined using a code for the medication no deeper than Level 3 of the ATC classification system. The ATC classification system is a well-established approach for classifying medications. By restricting the categories to be no deeper than Level 3 of the ATC classification system, consistent and interpretable data is made available for processing by the machine-learning algorithm.

In some examples, the step of processing each ATC code comprises identifying, for each ATC code, a Level 1 code, a Level 2 code and a Level 3 code of the ATC classification system for the ATC code.

The medication information may comprise a number of medications prescribed to and/or taken by the medical subject. These embodiments recognize that there is a strong correlation or link between number of medications and ASA-PS score. Thus, improved accuracy in determining the ASA-PS score can be achieved by using such information in calculating the ASA-PS score.

The medication information may comprise a square of the number of medications prescribed to and/or taken by the medical subject.

In some examples, the step of processing at least the medication information using a machine-learning method comprises: deriving at least one input feature from the medication information; inputting the at least one input feature to the machine-learning method; and outputting, from the machine-learning method, the ASA-PS score.

There is also proposed a computer-implemented method for training a machine-learning model for predicting an American Society of Anesthesiologists Physical Status, ASA-PS, score for a medical subject.

The computer-implemented method comprises: obtaining a training dataset comprising, for each of a plurality of historic medical cases: historic medication information identifying any medications prescribed and/or taken by a historic medical subject of the historic medical case; and a historic ASA-PS score for the medical subject of the historic medical case. The method also comprises training the machine-learning method using the training dataset.

There is also proposed a computer program product comprising computer program code means which, when executed on a computing device having a processing system, cause the processing system to perform all of the steps of any herein disclosed method.

There is also proposed a processing system for predicting an American Society of Anesthesiologists Physical Status, ASA-PS, score for a medical subject. The processing system is configured to obtain medication information identifying any medications prescribed and/or taken by the medical subject; and process at least the medication information using a machine-learning method to predict the ASA-PS score for the medical subject.

The processing system may be appropriately adapted to carry out the functions of any herein disclosed method, and vice versa.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Fig. 1: illustrates correlations between potential input features and an ASA-PS score;
- Fig. 2: illustrates a proposed method;
- Fig. 3: illustrates a further proposed method;
- Fig. 4: illustrates another proposed method;
- Fig. 5: illustrates a method of training a machine-learning algorithm; and
- Fig. 6: illustrates a method for use in embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for predicting an ASA-PS score for a medical subject. Medication information of the subject is processed using a machine-learning method to predict the ASA-PS score.

The present disclosure proposes a technique to predict an ASA-PS score or ASA-PS value for a medical subject. It is common for an anesthesiologist to perform the assessment of the ASA-PS value before procedures in which the medical subject undergoes anesthesia. However, in some cases, the value of ASA-PS is missing (e.g., from the medical record) due to lack of hospital staff or lack of inclusion/use of this measure by the clinical setting (e.g., hospital) themselves.

The proposed approach can be used to predict the ASA-PS score. This can be used to generate an ASA-PS score where none was previously available and/or act as a second or supplementary recommendation tool for a clinician, e.g., to identify one or more features responsible for the overall value prediction for the medical subject. The output of the proposed approach can thereby help the clinician(s) with decision making in handling possible complications and how best to manage them.

In particular, the present disclosure proposes an approach that uses a machine-learning method to process medication information identifying any medications prescribed and/or taken by the medical subject. The machine-learning method outputs a predicted ASA-PS score for the medical subject.

To achieve accurate prediction using the machine-learning method, there should be a correlation between the input feature(s) and the ASA-PS score. The present invention recognizes that there is a strong correlation between medication information and ASA-PS score. Further embodiments make use of the further recognition that there is a correlation between demographic information and/or renal state information and the ASA-PS score.

It is noted that the Anatomical Therapeutic Chemical (ATC) classification system is a well-established medication classification system maintained by the World Health Organization (WHO). The ATC classification system classifies medications (i.e., drugs or other active substances) at five different levels (i.e., starting at Level 1 and ending at Level 5), each level being progressively more specific.

It is also noted that the ASA-PS score makes use of a well-established scoring or classification system for defining the condition of the subject. In particular, an ASA-PS score defines a classification or score for the patient using the ASA Physical Status Classification System established by the American Society of Anesthesiologists (ASA).

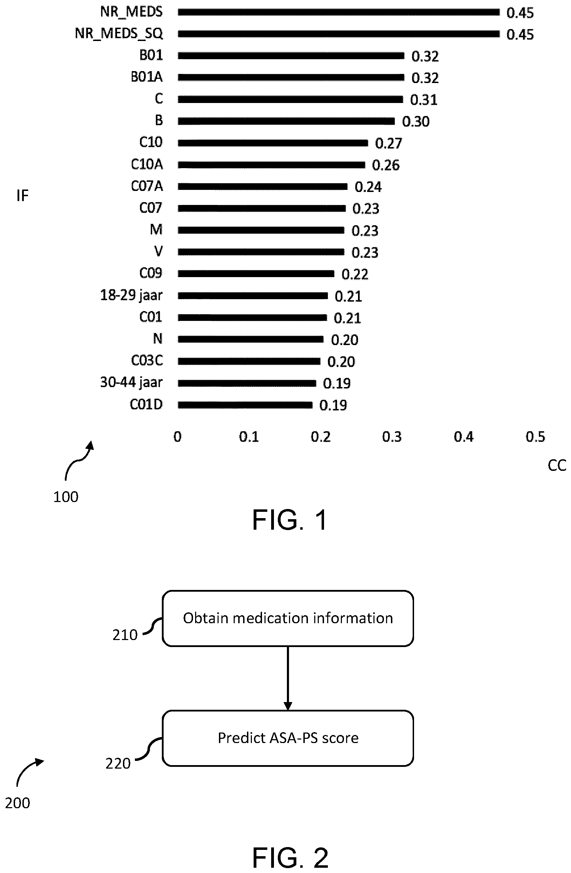

Fig. 1 is a bar chart 100 that illustrates correlation coefficients CC for a number of potential input features IF for a machine-learning method for predicting an ASA-PS score. The correlation coefficient represents the correlation between the relevant input feature and the ASA-PS score.

Each potential input feature may be defined using a numeric, categorical and/or binary data format. Each potential input feature represents a piece of medical information and/or a piece of demographic information and/or a piece of renal state information.

One example input feature is a number of medications prescribed to and/or taken by the medical subject NR_MEDS. Another example input feature is a square of the number of medications prescribed to and/or taken by the medical subject NR_ MEDS_SQ. Fig. 1 illustrates how there is a strong positive correlation between number of medications and ASA-PS score.

Another example input feature is a type of medication prescribed to and/or taken by the medical subject. Each of a plurality of different (e.g., predetermined) types of medications may each define an input feature. In particular, a plurality of different Anatomical Therapeutic Chemical (ATC) codes may each define a different input feature in binary format (i.e., identifying whether or not a medical subject is prescribed and/or taking a medication having that ATC code). As illustrated, each input feature for an ATC code may represent an ATC code that is no deeper than Level 3 of the ATC classification system. Example ATC codes illustrated in Fig. 1 include: B01; B01A; C; B; C10; C10A; C07A; C07; M; V; C09; C1; N; C03C; and C01D. These each represent well known and defined groups of medications.

Another example input feature is an age of the medical subject, here defined as an age group into which the medical subject falls. Each age group may be defined as a range of ages, and may be in a binary format (e.g., identifying whether or not the medical subject falls within that age group. Two example age groups, and their correlation coefficients, are illustrated: "18-29 jaar" and "30-44 jaar".

Fig. 1 illustrates how there is a positive correlation or relationship between certain input features and the ASA-PS score. The present disclosure makes use of these correlations to perform predictive determination of the ASA-PS score. In particular, a machine-learning method processes at least medication information of a medical subject in order to predict an ASA-score of the medical subject.

Fig. 2 is a flowchart illustrating a method 200 according to a proposed approach. The method 200 is configured for predicting an American Society of Anesthesiologists Physical Status, ASA-PS, score for a medical subject. The method 200 is a computer-implemented method, and may be carried out by a suitably configured processing system.

The method 200 comprises a step 210 of obtaining medication information identifying any medications prescribed and/or taken by the medical subject.

The medication information may, for instance, be stored in a medical record for the medical subject. Thus, the medication information may be stored in a database or other memory storage device. Step 210 may, for instance, comprise retrieving the medication information from the memory storage device, e.g., from a medical record of the subject stored in the medical storage device.

In some examples, the medication information may be input at a user interface. Thus, a user or individual may be able to define at least the medication information for the subject.

Suitable examples for the content of medical information have been previously described in the context of Fig. 1.

In particular, the medical information may contain a number (or a square thereof) of medications prescribed to and/or taken by the medical subject. There is a strong positive correlation between the number of medications for the medical subject and ASA-PS score. Use of a number of medications thereby facilitates more accurate prediction of the ASA-PS score.

In some examples, the medication information may comprise at least one ATC code for the medical subject. As previously explained, an ATC code may identify a medication prescribed and/or taken by the medical subject.

More particularly, the medication information may indicate (or be processable to identify), for each of a plurality of ATC codes, whether or not the medical subject is prescribed and/or taking a medication that falls within the category identified by the ATC code. As exemplified by

Fig. 1, there is a strong correlation between whether or not an individual is taking/prescribed medications of particular categories and ASA-PS score. Use of such medication information thereby facilitates more accurate prediction of the ASA-PS score.

In some scenarios, the medical information identifies one or ATC codes for the medical subject. Step 210 may comprise processing each ATC code to identify one or more categories of medication prescribed to and/or taken by the medical subject; and processing the identified categories of medication using the machine-learning method to predict the ASA-PS score for the medical subject.

Each category may be defined using a code for the medication no deeper than Level 3 of the ATC classification system. Thus, one or more high level categories for each medication can be effectively identified.

In some examples, the step of processing each ATC code comprises identifying, for each ATC code, a Level 1 code, a Level 2 code and a Level 3 code of the ATC classification system for the ATC code.

The method 200 also comprises a step 220 of processing at least the medication information using a machine-learning method to predict the ASA-PS score for the medical subject.

The ASA-PS score may be or have a categorical data format, e.g., identifying a category for the ASA-PS score. In particular, the ASA-PS score may identify in which of up to six categories (e.g., labelled 1 to 6) the medical subject is predicted to belong.

These categories are typically characterized as: (1) Healthy person; (2) Mild systemic disease; (3) Severe systemic disease; (4) Severe systemic disease that is a constant threat to life; (5) a moribund person who is not expected to survive without treatment (e.g., the surgery); and (6) declared brain-dead person whose organs are being removed for donor purposes.

It is known for category (6) to be omitted in some use-case scenarios. In such cases, the ASA-PS score may identify in which of up to 5 categories (labelled 1 to 5) the medical subject is predicted to belong.

A machine-learning algorithm is any self-training algorithm that processes input data in order to produce or predict output data. Here, the input data comprises at least medication information for a medical subject and the output data comprises an ASA-PS score for the medical subject. The input data may include other pieces of data or information, as later described.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of medication information. The training input data entries may further comprise corresponding instances of demographic information, renal state information and/or other information about the medical subject(s) (associated with the medication information). The training output data entries correspond to ASA-PS scores.

A more detailed approach for training a suitable machine-learning algorithm will be provided later in this disclosure.

Accordingly, it will be appreciated that step 210 can effectively comprise deriving at least one input feature from the medication information; inputting the at least one input feature to the machine-learning method; and outputting, from the machine-learning method, the ASA-PS score.

In some examples, the machine-learning algorithm is an ensemble of classification and regression algorithms. Such an algorithm has been identified as being particularly useful to identify or determine an ASA-PS score.

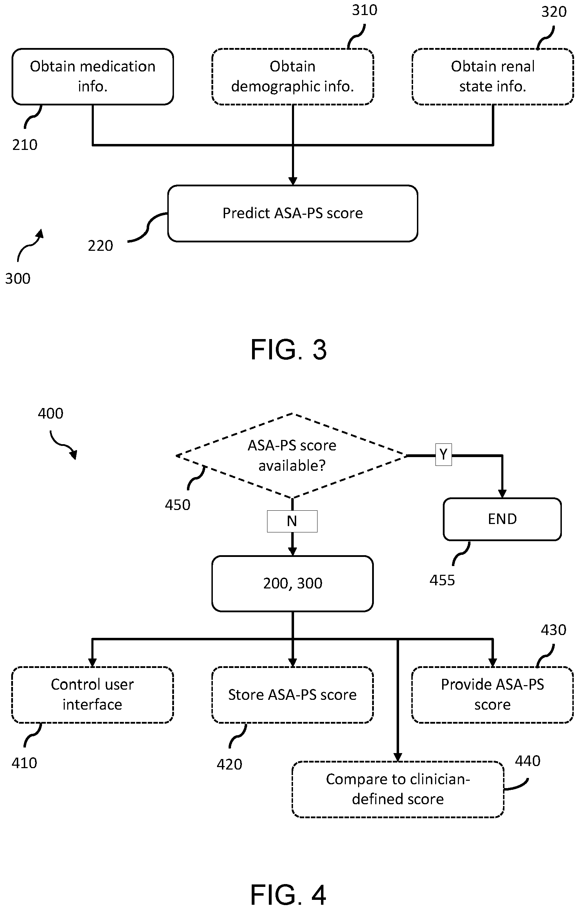

Fig. 3 is a flowchart illustrating further optional improvements to a method 300 for predicting an American Society of Anesthesiologists Physical Status, ASA-PS, score for a medical subject. As before, the method 300 is computer-implemented and may be carried out by a processing system.

The method 300 comprises steps 210 and 220 previously described.

The method 300 may further comprise a step 310 of obtaining demographic information of the medical subject. Accordingly, step 220 may be adapted to comprise processing at least the demographic information and the medication information using the machine-learning method to predict the ASA-PS score.

Examples of suitable demographic information include an age and/or gender of the medical subject. These pieces of information have been identified as having a positive correlation with an ASA-PS score, thereby improving the predictive capability of the machine-learning method.

The demographic information may be stored in a medical record or other database (e.g., if relevant, in a same location as the medication information). Accordingly, step 310 may comprise retrieving the demographic information from a memory storage device, e.g., from a medical record of the subject stored in the medical storage device. In another example, the demographic information may be input at a user interface. Thus, a user or individual may be able to define at least the demographic information for the subject.

**TABLE 1**

| | | | Error | | |
|---|---|---|---|---|---|
| Training Set | R2 Score | Mean Absolute | Mean Square | Median Square | Max |
| Med | 0.522 | 0.438 | 0.319 | 0.332 | 1.904 |
| Med + Dem | 0.838 | 0.171 | 0.108 | 0.171 | 1.617 |

Table 1 illustrates the effect of including demographic information for improving the accuracy of the machine-learning method. In particular, Table 1 demonstrates the R2 score and various error scores for a machine-learning method that produces predicted ASA-PS scores (e.g., which are compared to ground-truth ASA-PS scores) for a set of training data. As demonstrated in Table 1, the R2 score is significantly improved, and all measures of error reduced, when both medication and demographic information (Med + Dem) is processed using the machine-learning method compared to when only medication information (Med) is processed.

**TABLE 2**

| | | | Error | | |
|---|---|---|---|---|---|
| Testing Set | R2 Score | Mean Absolute | Mean Square | Median Square | Max |
| Med | 0.421 | 0.474 | 0.361 | 0.374 | 2.185 |
| Med + Dem | 0.563 | 0.394 | 0.273 | 0.288 | 2.25 |

Table 2 illustrates a same scenario for a different set (a testing set) of data, which demonstrates much the same outcome as previously explained with reference to Table 1.

The method 300 may further comprise a step 320 of obtaining renal state information of the medical subject. Accordingly, step 220 may be adapted to comprise processing at least the medication information and the renal state information using the machine-learning method to predict the ASA-PS score.

Of course, when steps 310 and 320 are performed, then step 220 may comprise processing at least the medication information, the demographic information and the renal state information using the machine-learning method to predict the ASA-PS score.

Examples of suitable renal state information includes information that identifies a change from normal functionality of the renal system of the medical subject. For instance, the renal state information may comprise a piece of categorical data that identifies a change in renal functionality, e.g., one of: "normal", "failure", "slightly decrease", "mild decrease" or "severe decrease" (or other data values representing each category).

These categories for renal state functionality of a medical subject have been identified as having a positive correlation with an ASA-PS score, thereby improving the predictive capability of the machine-learning method.

The renal state information may be stored in a medical record or other database (e.g., if relevant, in a same location as the medication information). Accordingly, step 310 may comprise retrieving the renal state information from a memory storage device, e.g., from a medical record of the subject stored in the medical storage device. In another example, the renal state information may be input at a user interface. Thus, a user or individual may be able to define at least the renal state information for the subject.

Other forms of information may also be obtained for processing by the machine-learning method. In particular, any other form of information that has a positive correlation with an ASA-PS score is a potential candidate for processing by the machine-learning method in predicting the ASA-PS score for the medical subject.

As an example, another form of information may be surgery information, e.g., identifying information of an upcoming or scheduled surgery for the medical subject (i.e., a surgery taking place after the time point for which the ASA-PS score is to be predicted). Surgery information may, for instance, identify one or more of: a type of surgery, a planned duration of surgery, a planned number of surgical staff and/or a number of surgical procedures to be performed.

It has been recognized that there is a positive correlation between surgery information and ASA-PS score. This is because the conditions of a scheduled surgery will be influenced by the condition of the medical subject, such that an ASA-PS score can be more accurately determined from surgery information.

Fig. 4 is a flowchart illustrating further optional steps for a proposed method 400. As before, the method 400 is computer-implemented and may be carried out by a processing system.

The method 400 comprises performing a previously described process 200, 300 for predicting an ASA-PS score.

The method 400 may comprise a step 410 of controlling a user interface to provide a visual representation of the predicted ASA-PS score. Thus, step 410 may comprise controlling a user interface to provide a user-perceptible visual output of the predicted ASA-PS score. Approaches for controlling a user interface (e.g., a screen or display) in this well are well established in the art. This provides a user or individual with useful information for understanding the state or condition of the medical subject with the predicted ASA-PS score, e.g., increasing their knowledge of the medical subject to facilitate improved clinical decision making.

By way of example, the predicted ASA-PS score may be used to make a decision on planning an upcoming surgical procedure (e.g., to account for any risks or the like), assessing the preferred length of observation for the medical subject (e.g., to reduce risk of deterioration), determining any additional medical screening and/or tests to perform on the medical subject and so on.

The method 400 may comprise a step 420 of storing the predicted ASA-PS score, e.g., in a medical record for the medical subject. This allows for later reference of predicted score (e.g., for comparison to other predicted and/or clinician-determined ASA-PS scores) to assess the condition of the subject and/or any changes to the condition of the medical subject.

The method 400 may comprise a step 430 of providing the predicted ASA-PS score to a further processing system. The further processing system may use the predicted ASA-PS score as an input for making a clinical prediction or generating a value for another parameter for the medical subject (e.g., a risk of deterioration, a risk of sepsis and so on). Other suitable uses for an ASA-PS score by a further processing system will be readily apparent to the appropriately skilled person.

The method 400 may comprise a step 440 of comparing the predicted ASA-PS score to a clinician-defined ASA-PS score. Step 440 may, for instance, comprise determining a difference between the predicted ASA-PS score and the clinician-defined ASA-PS score. In some examples, step 440 comprises generating a user-perceptible alert (e.g., a flashing light, a sound or a haptic alert) responsive to a difference between the predicted ASA-PS score and the clinician-defined ASA-PS score exceeding a predetermined value (e.g., 1). This approach provides a clinician with an indication that there is a potential error in the determination of the clinician-defined ASA-PS score and/or an unusual characteristic of the medical subject (causing the difference). This reduces a risk of error and/or identifies when a medical subject does not conform to expected characteristics.

In some examples, the method 400 may comprise a step 450 of determining whether or not an ASA-PS score for the medical subject is available, e.g., has been prepared by a clinician in advance of an upcoming medical procedure. As an example, step 450 may comprise determining whether an ASA-PS score has been produced within a predetermined time window (e.g., 1 hour, 2 hours) before a medical procedure, such as a surgery. Preferably, the medical procedure is one that requires or is scheduled to require anesthesia of the medical subject.

In some examples, the process 200, 300 for predicting an ASA-PS score is only performed in response to a positive determination in step 450. Otherwise, the ASA-PS score is not predicted, e.g., using process 200, 300, Rather, the clinician-defined ASA-PS score can be used (e.g., presented to a user, stored and/or further processed). Thus, the method may end in step 455 responsive to a negative determination in step 450.

Of course, process 200, 300 may be performed in any event responsive to a request or override received from a user, e.g., the clinician. This approach can be useful to compare the predicted score to a clinician-defined score, e.g., to reduce a risk of error by the clinician in defining the score. A large discrepancy between the clinician-defined score and the predicted score may indicate an error by the clinician and/or unusual characteristics of the medical subject that may have an impact on an upcoming procedure.

Any one or more of steps 410, 420, 430, 440 and/or 450 (and 455 where relevant) may be performed. Other suitable uses for the ASA-PS score will be apparent to the skilled person.

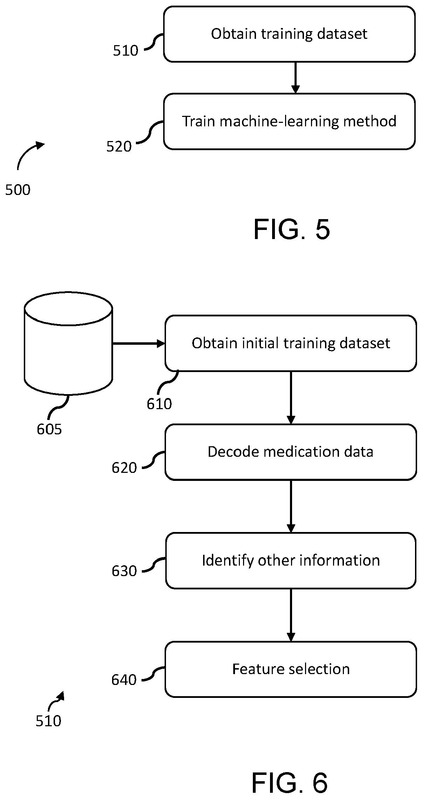

Fig. 5 is a flowchart illustrating a method 500 for training a machine-learning model for predicting an American Society of Anesthesiologists Physical Status, ASA-PS, score for a medical subject. The method 500 is computer-implemented and may be carried out by a processing system.

The method 500 comprises a step 510 of obtaining a training dataset. The training dataset comprises, for each of a plurality of historic medical cases, historic medication information identifying any medications prescribed and/or taken by a historic medical subject of the historic medical case; and a historic ASA-PS score for the medical subject of the historic medical case.

The method 500 also comprises a step 520 of training the machine-learning method using the training dataset.

Approaches for training a machine-learning method using a training dataset containing example input data and ground-truth output data are well known in the art. An example approach has been previously described.

Fig. 6 illustrates an example approach for performing step 510 for obtaining the training dataset. However, other approaches will be apparent to the skilled person.

The step 510 may comprise a sub-step 610 of obtaining an initial training dataset from a memory or storage unit 605. The initial training dataset may comprise, for each of a plurality of historic medical cases, a historic ASA-PS score and additional information for the medical subject of the historic medical case, e.g., including at least additional medical information.

The step 510 may comprise a sub-step 620 of, if necessary, decoding medication information from the additional information. Sub-step 620 may comprise, for each historic medical case, processing one or more ATC codes contained in the additional medical information to identify one or more categories of any medications prescribed or taken by the corresponding medical subject. This can be performed, for instance, using an online dictionary source using a scribing routine or the like. Each category may, for instance, be defined using a code for the medication no deeper than Level 3 of the ATC classification system. By way of example, each ATC code may be processed to identify, for each ATC code, a Level 1 code, a Level 2 code and a Level 3 code of the ATC classification system for the ATC code.

The step 510 optionally then performs a process 630 of identifying any other information for each historic medical case. Examples of other information include demographic information (e.g., an age, age range or gender) and/or renal state information. Other examples will be apparent to the skilled person.

Thus, steps 620 and 630 (if performed) effectively extract or derive data for potential features to be provided as input for a machine-learning method to produce a predicted ASA-PS score.

The step 510 may then perform a process 640 of feature selection. Feature selection comprises identifying features having a positive correlation with ASA-PS score. This can be performed by processing the initial training dataset to identify any correlations between potential features derived from the additional information and the historic ASA-PS scores. By way of example, features having a correlation coefficient above a predetermined value may be identified.

The features identified in step 640 can then be used to define the input features for the machine-learning method. The machine-learning method can subsequently be trained in step 520 using the initial training data and/or the data derived from the initial training dataset. In this way, the training dataset may be defined by, for each historic medical case, the historic ASA-PS score and data derived from the additional information for the features identified in step 640.

The skilled person would be readily capable of developing a processing system for carrying out any herein described method. Thus, each step of the flow chart may represent a different action performed by a processing system, and may be performed by a respective module of the processing system.

There is therefore provided a processing system for predicting an American Society of Anesthesiologists Physical Status, ASA-PS, score for a medical subject.

The processing system is configured to obtain medication information identifying any medications prescribed and/or taken by the medical subject; and process at least the medication information using a machine-learning method to predict the ASA-PS score for the medical subject.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method for predicting an American Society of Anesthesiologists Physical Status, ASA-PS, score for a medical subject, the computer-implemented method comprising:
obtaining medication information identifying any medications prescribed and/or taken by the medical subject; and
processing at least the medication information using a machine-learning method to predict the ASA-PS score for the medical subject.

2. The computer-implemented method of claim 1, further comprising obtaining demographic information of the medical subject, wherein processing at least the medication information comprises processing at least the demographic information and the medication information using the machine-learning method to predict the ASA-PS score.

3. The computer-implemented method of claim 2, wherein the demographic information comprises at least an age and/or gender of the medical subject.

4. The computer-implemented method of any of claims 1 to 3, further comprising obtaining renal state information indicating a renal state functionality of the medical subject, wherein processing at least the medication information comprises processing at least the renal state information and the medication information using the machine-learning method to predict the ASA-PS score.

5. The computer-implemented method of claim 4, wherein the renal state information identifies a change from normal functionality of the renal system of the medical subject.

6. The computer-implemented method of any of claims 1 to 5, wherein the medication information comprises at least one Anatomical Therapeutic Chemical, ATC, code for the medical subject, each ATC code identifying a medication prescribed and/or taken by the medical subject.

7. The computer-implemented method of claim 6, wherein the step of processing at least the medication information using the machine-learning method comprises:
processing each ATC code to identify one or more categories of medication prescribed to and/or taken by the medical subject; and
processing the identified categories of medication using the machine-learning method to predict the ASA-PS score for the medical subject.

8. The computer-implemented method of claim 7, wherein each category is defined using a code for the medication no deeper than Level 3 of the ATC classification system.

9. The computer-implemented method of claim 7 or 8, wherein the step of processing each ATC code comprises identifying, for each ATC code, a Level 1 code, a Level 2 code and a Level 3 code of the ATC classification system for the ATC code.

10. The computer-implemented method of any of claims 1 to 9, wherein the medication information comprises a number of medications prescribed to and/or taken by the medical subject.

11. The computer-implemented method of claim 10, wherein the medication information comprises a square of the number of medications prescribed to and/or taken by the medical subject.

12. The computer-implemented method of any of claims 1 to 11, wherein the step of processing at least the medication information using a machine-learning method comprises:
deriving at least one input feature from the medication information;
inputting the at least one input feature to the machine-learning method; and
outputting, from the machine-learning method, the ASA-PS score.

13. A computer-implemented method for training a machine-learning model for predicting an American Society of Anesthesiologists Physical Status, ASA-PS, score for a medical subject, the computer-implemented method comprising:
obtaining a training dataset comprising, for each of a plurality of historic medical cases:
historic medication information identifying any medications prescribed and/or taken by a historic medical subject of the historic medical case; and
a historic ASA-PS score for the medical subject of the historic medical case, training the machine-learning method using the training dataset.

14. A computer program product comprising computer program code means which, when executed on a computing device having a processing system, cause the processing system to perform all of the steps of the computer-implemented method according to any of claims 1 to 13.

15. A processing system for predicting an American Society of Anesthesiologists Physical Status, ASA-PS, score for a medical subject, the processing system being configured to:
obtain medication information identifying any medications prescribed and/or taken by the medical subject; and
process at least the medication information using a machine-learning method to predict the ASA-PS score for the medical subject.
